(19) **Europäisches Patentamt**
**European Patent Office**
**Office européen des brevets**

(11) Veröffentlichungsnummer : **0 463 138 B1**

(12) **EUROPÄISCHE PATENTSCHRIFT**

(45) Veröffentlichungstag der Patentschrift :
**11.05.94 Patentblatt 94/19**

(21) Anmeldenummer : **91902160.0**

(22) Anmeldetag : **04.01.91**

(86) Internationale Anmeldenummer :
**PCT/EP91/00004**

(87) Internationale Veröffentlichungsnummer :
**WO 91/10449 25.07.91 Gazette 91/17**

(51) Int. Cl.[5] : **A61K 39/395,** A61K 45/06,
// (A61K39/395, 37:38),
(A61K39/395, 31:135)

(54) **ARZNEIMITTEL ZUR VERBESSERUNG DER OVARREAKTIONEN UND DER EIZELL- UND EMBRYONEN-PRODUKTION BEI HAUSSÄUGETIEREN IN VERBINDUNG MIT BIOTECHNOLOGICHEM EMBRYONENTRANSFER.**

(30) Priorität : **08.01.90 DE 4000327**

(43) Veröffentlichungstag der Anmeldung :
**02.01.92 Patentblatt 92/01**

(45) Bekanntmachung des Hinweises auf die
Patenterteilung :
**11.05.94 Patentblatt 94/19**

(84) Benannte Vertragsstaaten :
**AT BE CH DE DK ES FR GB IT LI NL**

(56) Entgegenhaltungen :
**EP-A- 0 276 166**
**WO-A-86/00078**
**US-A- 4 864 019**
**CHEMICAL ABSTRACTS, Band 112, Nr. 1, 01 Januar 1990, Columbus, OH (US); G.E. MANN et al., Seite 90, Nr. 915h**

(56) Entgegenhaltungen :
**BIOLOGICAL ABSTRACTS, Band 82, Nr. 10, 1986, Philadelphia, PA (US); L.J. CUMMINS et al., Seite AB-1026, Nr. 97579**
**CHEMICAL ABSTRACTS, Band 112, Nr. 15, 09 April 1990, Columbus, OH (US); M. MIZUMACHI et al., Seite 89, Nr. 132566e**
**CHEMICAL ABSTRACTS, Band 112, Nr. 9, 26 Februar 1990, Columbus, OH (US); J.H.M. WRATHALL et al., Seite 106, Nr. 70202x**

(73) Patentinhaber : **Heiden Stefan**
**Hofgut Himmigerode 1**
**D-37130 Gleichen/Sattenhausen (DE)**

(72) Erfinder : **Heiden Stefan**
**Hofgut Himmigerode 1**
**D-37130 Gleichen/Sattenhausen (DE)**

(74) Vertreter : **Jahn-Held, Wilhelm W. Dr.Dr.-Ing. Dipl.-Chem.**
**Schöne Aussicht 8**
**D-34355 Staufenberg (DE)**

EP 0 463 138 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt für Säugetiere, die innerhalb eines Fortpflanzungszyklus in der Regel natürlicherweise nur eine befruchtete Eizelle produzieren (monotoke Tierarten), eine Steigerung der Produktion von Eizellen und daraus von Embryonen durch Einsatz von Gonadotropinen, wie beispielsweise HCG, HMG, PMSG, FSH, LH und/oder GnRH, das die Freisetzung von Gonadotropinen aus der Hypophyse steuert, zu bewirken.

Es ist dadurch möglich, hintereinander eine Serie von befruchtungsfähigen Eizellen zu produzieren und diese nach der Befruchtung als Embryonen zu gewinnen und auf Empfängertiere zu transferieren. Diese Stimulationsmethode zur Erzielung von Superovulationen hat den Nachteil, dass diese nur durch Überdosierung von gonadotropen Hormonen erreicht werden kann. Aufgrund der endogenen Gegenregulation gegen Mehrfachovulationen besonders bei monotoken Haussäugetierarten resultieren aus solchen bekannten Stimulationsmethoden unbefriedigende Ovarreaktionen, insbesondere hinsichtlich der starken zwischen- und innerartlichen und individuellen Variabilität, sowie der daraus resultierenden Anzahl qualitativ hochwertiger Embryonen. Ein durch Überdosierung insbesondere von Gonadotropinen bedingter, erheblicher Mehrverbrauch von Oogenien und Oozyten führt zu einer Verminderung der potentiellen Lebensproduktionsleistung eines Spendertieres. Ein solcher starker Eingriff in die endogen endokrine Regulation von Follikulogenese, Follikelreifung und in die Ovulationsprozesse führt zu temporären und auch zu bleibenden Folgeschäden der Reproduktionsleistung von Spendertieren.

Diese Nachteile nach dem Stand der Technik werden durch das Arzneimittel der Erfindung vermieden.

Soweit nach dem Stand der Technik als Inhibin wirkende Stoffe eingesetzt wurden, erfolgte diese Verwendung als aktive Immunisierung oder zur Untersuchung der Auswirkungen auf physiologische Reproduktionsparameter. Soweit nach dem Stand der Technik als Inhibin- neutralisierender Antikörper wirkende Stoffe eingesetzt wurden, erfolgte diese Verwendung zum diagnostischen Nachweis und zur Erzielung einer Profertilitätswirkung bei individuellen, weiblichen Säugetieren.

Fertilität bezeichnet die Fähigkeit zur Fortpflanzung eines Individuums und die Kapazität des Individuums, eine bestimmte Anzahl lebender Nachkommen je Fortpflanzungsperiode hervorzubringen.

Eine Einsetzung zur Herbeiführung von Superovulation ist dagegen keine Fruchtbarkeits- fördernde Massnahme.

Die WO/ 8600078 beschreibt die Gewinnung von Inhibin und dessen therapeutische Anwendung zur Erzielung eines Profertilitätseffektes.

Dagegen ist es die Aufgabe des Arzneimittels der Erfindung dieses zur Erzielung konstanter Ovarreaktionen und eines höheren Anteiles transfertauglicher, qualitativ hochwertiger Embryonen einzusetzen.

Das Arzneimittel der Erfindung ist kein Mittel zur therapeutischen Verwendung, sondern ein Mittel zur Verwendung bei gesunden, fertilen weiblichen Tieren zur Produktion von Eizellen und Embryonen in Verbindung mit biotechnologischem Embryonentransfer.

Chemical Abstracts 112/1, 1990, Seite 90 beschreibt nur eine Erhöhung der Ovulationsrate um 0,4 pro behandeltes Tier, die zu einem , die Fruchtbarkeit fördernden Effekt führen soll. Es wird nur eine einmalige Injektion angewendet.

Dieser Stand der Wissenschaft hat nur dazu angeregt, reinere, spezielle Gonadotropine zu entwickeln.

Die US-A- 4 864 019 beschreibt die Isolation eines 32 kDalton- Proteins (Inhibin) aus porkiner Follikelflüssigkeit, das aus 2 Untereinheiten von 18 und 14 kDalton besteht, die durch Disulfidbrücken verknüpft sind, und welches spezifisch die FSH- Sekretion hemmt.

Es sollen polyklonale Antikörper das endogene Inhibin neutralisieren und dadurch die Gonadotropin- Sekretion fördern und zur Kontrolle der Fruchtbarkeit verwendet werden können.

Dieser Stand der Technik hat kein Anregung dazu gegeben, die Kombination der beanspruchten Stoffe einzusetzen, weil daraus keine Vorteile erwartet werden konnten.

Der durch das Arzneimittel der Erfindung erzielte, synergistische Effekt trägt unerwartet zur Steigerung der Trächtigkeitsrate durch Erzeugung transfertauglicher Embryonen bei.

Gegenstand der Erfindung ist ein Arneimittel zur Verbesserung der Ovarreaktionen und der Eizell- und Embryonen- Produktion bei Haussäugetieren in Verbindung mit biotechnologischem Embryonentransfer.

Es ist die Aufgabe des Arzneimittels der Erfindung durch gezielten Eingriff in die zentrale endogene endokrine Regulation des Ovulationsraten- Kontrollsystems die Superovulation einzuleiten und dadurch eine universellere Methode zur Superovulationsstimulation für verschiedene Haussäugetierarten zu entwickeln. Weiter ist es die Aufgabe der Erfindung konstantere Ovarreaktionen und einen höheren Anteil transfertauglicher, qualitativ hochwertiger Embryonen zu erzielen und dadurch die Wirtschaftlichkeit des Embryotransfers bei Haussäugetieren zu steigern.

Die Lösung der Aufgabe der Erfindung ist im kennzeichnenden Teil des Patentanspruches 1 definiert.

Das Arzneimittel der Erfindung ist alternativ und bevorzugt in den Unteransprüchen definiert.

Das Arzneimittel zur Verbesserung der Ovarreaktionen und der Eizell- und Embryonen- Produktion bei Haussäugetieren in Verbindung mit biotechnologischem Embryonentransfer ist dadurch gekennzeichnet, dass dieses aus Inhibin- neutralisierenden Antikörpern in Verbindung mit Mehrfachovulationen (Superovulation) fördernden Hormonen, vorzugsweise mit Gonadotropinen und/ oder mit GnRH (Gonadotropin-Releasing- Hormon, und / oder mit Antiöstrogenen in form von synthetischen Wirkstoffen oder Antikörpern, vorzugsweise Glomiphen, oder Tamoxifen, und/oder mit Antiandrogenen in Form von synthetischen Wirkstoffe oder Antikörpern, vorzugsweise gegen Androsteron, Testosteron, Dihydrotestoteron, besteht, welche Inhibin- neutralisierenden Antikörper gegen tierartlich unterschiedliches, und humanes, natürlich vorkommendes, oder rekombinantes (synthetisches) Inhibin oder seiner Untereinheiten, oder seiner Fragmente oder seiner Derivate gerichtet sind, und welches Arzneimittel die biologische Aktivität des in Gonaden gebildeten Inhibins aufheben kann und dadurch innerhalb eines Fortpflanzungszyklus multiples Follikelwachstum und multiple Follikelreifung bis zur Superovulation durch immunisierung bewirkt.

Das Arzneimittel der Erfindung ist auch dadurch gekennzeichnet, dass als Hormone Gonadotropine und-/oder GnRH ( Gonadotropin-Releasing- Hormone oder Antiöstrogene in Form von synthetischen Wirkstoffen oder Antikörper oder Antiandrogene in Form von synthetischen Wirkstoffen oder Antikörper gegen Androgene, wie Androstendion, Testosteron, Dihydrotestosteron eingesetzt werden.

Gegenstand der Erfindung ist auch ein Verfahren zur Verbesserung der Ovarreaktionen und der Einzell- und Embryonen- Produktion bei Haussäugetieren in Verbindung mit biotechnologischem Embryonentransfer unter Verwendung des Arzneimittels der Erfindung, dadurch gekennzeichnet, dass die Applikation als mehrmalige Injektion in definierten Zeitabständen von 12 oder 24 Stunden über mehrere, aufeinander folgende lage von 3,5 bis 5 Tagen, erfolgt.

Weiter ist dieses Verfahren dadurch gekennzeichnet, dass die Applikationen in der mittleren Lutealphase beginnend am 7. bis 11. Zyklustag erfolgt und dadurch dass die Appliaktionen in gleicher oder absteigender Dosierung mit je 2 gleichen, aufeinanderfolgenden Mengen erfolgt.

Die folgenden Autoren beschreiben die Charakterisierung eines Inhibin ( Antigen ) für die Tierart Rind.

Robertson,D.M, et all,Molecular and cellular Endocrinology, 44 (1986) 271-277.

Forage,R.G.,et all.Proc.Natl.Acad Sci., USA (1986) 3091-3095.

Es wird die Isolierung und Charakterisierung durch Klonierung und Sequenzanalyse von DNA, die für die Untereinheiten von Inhibin aus boviner Follikelflüssigkeit (bFF) kodieren, beschrieben.

Es wurde die 31 kDa- Form des Inhibins durch Einführung eines pH- 4,75- Präzipitationsschrittes in die beschriebene Reinigungsmethode für das 58 kDa-Inhibin aus bFF erzeugt. Dieses ist daher ein Spaltungsprodukt von 58 kDa-Inhibin.

Das Arzneimittel der Erfindung versteht unter Antikörpern solche, welche die biologische Aktivität der in Gonaden produzierten Inhibine im Tier neutralisieren und dadurch wirkungslos machen.

Miyamoto K. et all beschreiben in Biochemical and Biophysical Research communications,Vol.136,No.3 (1986), 1103 - 1109 Inhibin- Antikörper für die Tierart Rind. Dies sind monoclonale Antikörper gegen das bFF 32 kDa- Inhibin aus boviner Follikelflüssigkeit.

Monoclonale Antikörper sind gegen bFF 32 kDa- inhibin unter Anwendung von Hybridisierungstechnik in Mäusen (Stamm Balb/c) bekannt.

Ein bestimmter Antikörper, Typ 256 H, erkennt spezifisch die bovine 20 kDa ($\alpha$-Untereinheit) und 13 kDa ($\beta$- Untereinheit) von bovinem 32 kDa- Inhibin. Dieser weist im Vergleich zu anderen Antikörpern eine strikte Spezifität für die bovine 20 kDa- Untereinheit auf.

Das Arzneimittel nach Anspruch 1 ist bei den meisten Haussäugetieren bezüglich der Ovarreaktionen überlegen.

Die Verwendung des Arzneimittels der Erfindung nach Anspruch 1 wird beispielsweise wie folgt beschrieben.

Es wird die Wirkung der Kombination von Inhibin- neutralisierenden Antikörpern (Antiserum) in Verbindung mit Superovulationen förderden Hormonen ( Gonadotropin ) nachgewiesen.

Es stehen 15 Rinder der Rasse " Deutsche Schwarzbunte " mit etwa 75% " Holstein-Frisian " - Einkreuzung in den Altersklassen 17,5 bis 25 Monate und mit den Gewichtsklassen von etwa 350 bis 410 kg aus einem landwirtschaftlichen Betrieb zur Verfügung.

Diese Rinder wurden vorsynchronisiert mit 2- maliger Injektion von je 500 µg Chloprosterol der Handelsmarke " Estrumate " , Handelsprodukt der Coopers Tierarzneimittel GmbH, DE-3006 Burgwedel.

Die Injektionen erfolgten im Abstand von 11 Tagen. Danach wurden die Rinder nach dem Zufallsprinzip einer von 2 Gruppen zugeteilt.

Die Rinder befanden sich zu Beginn der Behandlung am 9. Zyklustag.

Es wurde den Rindern der Gruppe I, Kontrollgruppe, mit n = 8 Rindern, eine Gesamtdosis von 23,8 mg NIH- FSH- Standard- Äquivalent ( National Institute of Health- FSH) in 12-stündigem Abstand über 3,5 Tage in absteigender Dosierung mit 4,2 / 4,2/ 3,5 / 3,5 / 2,8 / 2,8 / 2,8 mg FSH- Äquivalent appliziert. Es wurde zusätzlich zur 6. und 7. Injektion je 500 μg Chloprostenol zur Einleitung der Luteolyse verabreicht. Unter Chloprostenol wird das wasserlösliche Natriumsalz verstanden.

Die künstliche Besamung erfolgte 36, 48, 60, 72 Stunden nach Einleiten der Luteolyse mit tiefgefrorenem Sperma eines einzigen Bullen.

Den Rindern der Gruppe II mit n = 7 Rindern wurde zusätzlich zu der vorstehend beschriebenen Behandlung zugleich mit den FSH- Injektionen je 20 ml eines mit 4 Schafen gepoolten Antiserums gegen Inhibin S-Antiserum i.v. injiziert.

Zur Erzeugung dieses Serums wurden 4 Schafböcke mit einer gereinigten Präparation von Inhibin aus boviner Follikelflüssigkeit, bFF, die auf einem Schlachthof von Rindern gewonnen wurde, aktiv immunisiert.

Die Rinder, aus deren Ovarien die Follikelflüssigkeit gewonnen wurde, sind vorher mit PMSG zur Erhöhung der Inhibin- Konzentration in der bFF behandelt worden.

Die Schafböcke wurden zunächst mit je 2,5 mg Protein einer gereinigten Inhibin- Präparation, gelöst in 2,0 ml einer 0,9 %-igen NaCl- Lösung und mit 2,0 ml kompletten Freundschen Adjuvanz grundimmunisiert.

Die Boosterungen erfolgten am 20. und 50. Tag nach der Grundimmunisierung mit je 2,0 und 1,8 mg Protein, gelöst in 2,0 ml 0,9 %-igen NaCl- Lösung und mit 2,0 ml inkomplettem Freundschem Adjuvanz.

Die Serumgewinnung erfolgte am 7. und 10. Tag nach der 2. Boosterung. Das Antiserum der 4 Schafböcke wurde zu je gleichen Volumenteilen gepoolt und fraktioniert, 150 ml, und bei - 20°C gelagert.

Es wurden folgende Ergebnisse erzielt :


Behandlung: Eizellen / Embryonen

| Komponenten | n | Cl | +/- | total |
|---|---|---|---|---|
| Folltropin 23,8 mg | 8 | 10,5 | 5,88 | 7,9 |
| Folltropin 23,8 mg + S- Antikörper gegen Inhibin | 7 | 14,1 | 3,29 | 9,6 |


Behandlung: Eizellen/Embryonen

| Komponenten | +/- | befruchtet | +/- | % | transfer-tauglich | +/- | % |
|---|---|---|---|---|---|---|---|
| Folltropin 23,8 mg | 4,76 | 5,1 | 3,27 | 66 | 3,8 | 3,06 | 48 |
| Folltropin 23,8 mg + S- Antikörper gegen Inhibin | 2,64 | 8,6 | 2,44 | 90 | 8,3 | 2,21 | 86 |

Die Zahlenwerte sind mit den mittleren Abweichungen ( Σn- 1 ) angegeben als Durchschnittswerte, die sich aus dem arithmetrischen Mittel der Summe der Einzelwerte der Tiere ergeben.

Folltropin ist eine aus Hypophysenzellen von Schlachtschweinen gewonnenen Gonadotropin- Präparation mit geringem LH- Anteil. Es wurden verwendet: Folltropin, Handelsprodukt der Vetrepharm Inc., London, Ontario. Vertrieb für DE: Praemix GmbH, Mannheim.

Es bedeuten in der Tabelle:

n         = Anzahl der Versuchstiere,
G1        =Gelbkörper, ermittelt durch rektale Palpation,als Merkmal der Anzahl erfolgter Ovulationen.
total     = Anzahl aller gewonnenen Eizellen, unbefruchtet und der Embryonen, % = auf " total " bezogene Zahlenwerte, Superovulation - förderndes Hormon = Folltropin, Handelsprodukt.

Die Behandlung mit Folltropin in der Gruppe I, Kontrollgruppe zeigt eine durchschnittliche Gewinnung von 7,9 Embryonen und Eizellen pro Einzelltier. Der Anteil befruchteter Embryonen beträgt 66 % (5,1) und der Anteil an transfertauglichen Embryonen 48 % (3,8).

Das Arzneimittel der Erfindung mit der Kombination von Antikörpern und Gonadotropinen führt zu einer unerwarteten und erheblichen Steigerung der Gewinnung von Eizellen und Embryonen auf 9,6 und des Anteiles der befruchteten Embryonen auf 90 % (8,6) und der transfertauglichen Embryonen auf 86 % (8,3).

Wenn eine durchschnittliche Trächtigkeitsrate aus Embryonentransfer von 60 % zugrunde gelegt wird, dann können aus 3,8 transfertauglichen Embryonen, Gruppe I, in der Praxis im Durchschnitt 2,3 Trächtigkeiten erwartet werden.

Die mit dem Arzneimittel der Erfindung erzeugten 8,3 transfertauglichen Embryonen führen dagegen unerwartet zu einer Steigerung auf zu erwartende 5,0 Trächtigkeiten.

Das Arzneimittel der Erfindung führt also überraschend zu einer Verdoppelung der Anzahl an Nachkommen eines Spendertieres gegenüber dem Stand der Technik.

Ein weiterer, praktischer Versuch wurde unter ähnlichen Bedingungen in einem anderen Betrieb mit Rindern durchgeführt.

Es standen 7 Rinder der gleichen Rasse in den Altersklassen von etwa 16 bis 19 Monaten und in den Gewichtsklassen von etwa 320 bis 360 kg zur Verfügung.

Die Synchronisierung, sowie die weitere Behandlung der Rinder erfolgte wie im ersten Versuch.

Die Rinder in Gruppe I, Komtrollgruppe mit n = 3, wurden abweichend von Versuch 1 mit einer Gesamtdosis von 16,9 mg NIH-FSH-standard- Äquivalent , Folltropin, in gleicher Weise behandelt. Die Einzeldosierungen betrugen 3,15 / 3,15 / 2,45 / 2,45/ 1,9 / 1,9 mg für die jeweilige Injektion.

Den Rinder in Gruppe II, Versuchsgruppe mit n = 4 Rindern, wurden zusätzlich zur Behandlung der Kontrollgruppe 1 zeitgleich mit den FSH- Injektionen je 15 ml eines von 6 Kaninchen gepoolten Antiserums gegen Inhibin i. v. injiziert.

Die Erzeugung des Kaninchen- Antiserums, K- Antiserum, gegen Inhibin erfolgte analog zur, bei den Schafböcken, angewandten Methode.

Es wurden folgende Ergebnisse erzielt :

```
Behandlung: Eizellen/ Embryonen

Komponenten   n  Cl    +/-  total


Folltropin    3  14,7 4,9  5,3
16,9 mg


Folltropin    4  17,0 1,4  11,8
16,9 mg +
K- Antiserum
gegen Inhibin
```

Eizellen/Embryonen

| Komponenten | +/- | befruchtet | +/- | % | transfer-tauglich | +/- | % |
|---|---|---|---|---|---|---|---|
| Folltropin 16,9 mg | 4,2 | 4,0 | 3,6 | 75 | 2,0 | 2,0 | 38 |
| Folltropin 16,9 mg + K- Antiserum gegen Inhibin | 2,2 | 10,3 | 1,3 | 87 | 6,5 | 1,9 | 63 |

Das Arzneimittel der Erfindung mit der Kombination von Antikörpern und Gonadotropinen führt auch in diesem Versuch zu einer erheblichen Steigerung der durchschnittlich gewonnenen Eizellen und Embryonen von 5,3 auf 11,8, sowie einer erheblichen Steigerung des Anteiles befruchteter Embryonen von 75 % (4,0) auf 87 % (10,3) und der transfertauglichen Embryonen von 38 % (2,0) auf 63 % (6,5).

Bei einer erzielbaren Trächtigkeitsrate von 60 % aus Embryonentransfer können aus 2,0 transfertauglichen Embryonen durchschnittlich 1,2 Trächtigkeiten erwartet werden.

Die Anwendung des Arzneimittels der Erfindung führt dagegen mit 6,5 transfertauglichen Embryonen pro Spendertier zu einer Steigerung auf 3,9 zu erwartende Trächtigkeiten. Es wird damit das Ergebnis des ersten Versuches unabhängig bestätigt. Dieses Ergebnis ist überraschend.

Die Versuche wurden vom Erfinder durchgeführt.

Es kann auch noch folgendes, überraschende Ergebnis aus den Versuchen festgestellt werden: In beiden Versuchen ist die mittlere Abweichung der Einzelwerte vom Mittelwert (Durchschnittswert) bei höheren Absolutwerten aller untersuchten Parameter in den mit dem Arzneimittel der Erfindung behandelten Gruppen erheblich geringer als in den Kontrollgruppen. Eine Verbesserung der Stimmulation erfolgt insbesondere auch durch konstantere Ovarreaktionen des Arzneimittels der Erfindung.

Der Einsatz von Inhibin neutralisierenden Antikörpern erfolgt nach dem Stand der Technik durch Einzelinjektion am 2.oder am 8. Zyklustag.

Dies bestätigen die folgenden Veröffentlichungen: J.H.M. Wrathall et all, Journal of Endocrinology (1990) 124,167-176. Siehe Seite 169,rechte Spalte, unter " Experiment 2",,Zeile 7 - 9:"On day 2". G.E.Mann et all. , l.c., (1989) 123, 383-391. Siehe Seite 385, linke Spalte, unter " Immunization experiment", Zeile 15: " " On day 8 of the luteal phase".

Das Arzneimittel der Erfindung wird dagegen wie folgt appliziert: Es erfolgen mehrmalige Injektionen in definierten Zeitabständen, vorzugsweise in 12 oder 24 Stunden über mehrere, aufeinander folgende Tage, vorzugsweise zwischen 3,5 und 4 Tagen.

Die Behandlung erfolgt im Allgemeinen am optimalsten in der Lutealphase, vorzugsweise vom 7. bis 11. Zyklustag. In diesem Intervall tritt auf den Ovarien eine neue Welle wachsender Follikel auf.

Ein ähnliches Behandlungsschema wird beim Embryonentransfer bei der Behandlung mit Gonadotropin angewendet.

Das Arzneimittel der Erfindung bietet den technischen Vorteil einer gesteigerten Produktion von qualitativ hochwertigen, transfertauglichen Embryonen pro Spülung eines Spendertieres bei erheblich konstanteren Ovarreaktionen.

Die dadurch erhöhte Produktion an wertvollen Nachkommen bewirkt eine erhebliche Steigerung der Wirtschaftlichkeit von Embryonentransfer- Programmen (ET-Programmen), insbesondere bei Rindern.

Das Arzneimittel der Erfindung bietet den weiteren Vorteil, dass seine Anwendung auch bei solchen Tierarten möglich ist, die bisher nicht oder nur mit geringem Erfolg stimuliert werden können, insbesondere bei Stuten.

**Patentansprüche**

1. Arzneimittel zur Verbesserung der Ovarreaktionen und der Eizell- und Embryonen- Produktion bei Haussäugetieren in Verbindung mit biotechnologischem Embryonentransfer, dadurch gekennzeichent, dass dieses aus Inhibin- neutralisierenden Antikörpern in Verbindung mit Mehrfachovulationen(Superovulation) fördernden Hormonen, vorzugsweise mit Gonadotropinen und/ oder mit GnRH (Gonadotropin-Releasing-Hormon, und / oder mit Antiöstrogenen in Form von synthetischen Wirkstoffen oder Antikörpern, vorzugsweise Glomiphen, oder Tamoxifen, und/oder mit Antiandrogenen in Form von synthetischen Wirkstoffe oder Antikörpern, vorzugsweise gegen Androsteron, Testosteron, Dihydrotestoteron, besteht, welche Inhibin-neutralisierenden Antikörper gegen tierartlich unterschiedliches, und humanes, natürlich vorkommendes, oder rekombinantes (synthetisches) Inhibin oder seiner Untereinheiten, oder seiner Fragmente oder seiner Derivate gerichtet sind, und welches Arzneimittel die biologische Aktivität des in Gonaden gebildeten Inhibins aufheben kann und dadurch innerhalb eines Fortpflanzungszyklus multiples Follikelwachstum und multiple Follikelreifung bis zur Superovulation durch Immunisierung bewirkt.

2. Arzneimittel nach Anspruch 1, dadurch gekennzeichnet, dass als Hormone Gonadotropine und/oder GnRH (Gonadotropin-Releasing-Hormone) eingesetzt werden.

3. Arzneimittel nach Anspruch 1, dadurch gekennzeichnet, dass als Hormone Antiöstrogene in Form von synthetischen Wirkstoffen oder Antikörpern eingesetzt werden.

4. Arzneimittel nach Anspruch 1, dadurch gekennzeichnet, dass als Hormone Antiandrogene in Form von synthetischen Wirkstoffen oder Antikörper gegen Androgene, wie Androstendion, Testosteron, Dihydrotestosteron, eingesetzt werden.

5. Verfahren zur Verbesserung der Ovarreaktionen und der Eizell- und Embryonen- Produktion bei Haussäugetieren in Verbindung mit biotechnologischem Embryonentransfer unter Verwendung des Arzneimittels nach den Ansprüchen 1 bis 4, dadurch gekennzeichnet,
dass die Applikation als mehrmalige Injektion in definierten Zeitabständen von 12 oder 24 Stunden über mehrere, aufeinander folgende Tage von 3,5 bis 5 Tagen, erfolgt.

6. Verfahrennach Anspruch 5, dadurch gekennzeichnet, dass die Applikation in der mittleren Lutealphase beginnend am 7. bis 11. Zyklustag erfolgt.

7. Verfahren nach Anspruch 6, dadurch gekennzeichnet, dass die Applikation in gleicher oder absteigender Dosierung mit je 2 gleichen, aufeinander folgenden Mengen erfolgt.

**Claims**

1. A compound for the improvement of ovary reactions and for the ova and empryo production in domestic mammals in combination with biotechnological embryo transfer, characterized in that the compound consists of inhibin-neutralizing antibodies in combination with hormones propagating multiple ovulations (superovulation) preferably with gonadotropenes and/or with GnRH (gonadotrpin-releasing hormone), and/or with antiestrogenes in the form of synthetic active materials or antibodies, preferably clomiphene, or tamoxifene, and/or with antiandrogenes in the form of synthetic active materials or antibodies, preferably against androsteron, testosteron, dihydrotestosteron, wherein said inhibin-neutralizing antibodies are directed against species-differing and human, naturally occurring or recombinant (synthetic) inhibin or its subunits, or its fragments or its derivatives, and which compound neutralizes the biological activity of inhibin which is formed in gonads, and thereby, within a reproduction cycle, causes through immunization multiple follicular growth and multiple follicle ripening up to multiple ovulation (superovulation) in mammals.

2. A compound according to Claim 1, characterized in that gonadotropenes and/or GnRH (gonadotropin-releasing hormone) are used as hormones.

3. A compound as claimed in Claim 1, characterized in that antiestrogenes in the form of synthetic active materials or antibodies are used as hormones.

4. A compound as claimed in Claim 1, characterized in that antiandrogenes in the form of synthetic active materials or antibodies, which are directed against androgenes such as androsteron, testosteron, dihydrotestosteron, are used as hormones.

5. A method for the improvement of ovary reactions and the ova and embryo production in domestic mammals in combination with biotechnological embryotransfer by utilization of the compounds according to Claims 1 through 4, characterized in that the administration is carried out in multiple injections at defined time intervals, preferably 12 or 24 hours over a number of successively following days, preferably of 3.5 to 5 days.

6. A method according to Claim 5, characterized in that the administration is carried out during the middle luteal phase, preferably on the 7th to the 11th cycle.

7. A method according to Claim 6, characterized in that the administration is carried out in equal or decreasing dosages with presently two eqal successively following quantities.


**Revendications**

1. Médicament destiné à améliorer les réactions ovariennes et la production d'ovules et d'embryons chez le mammifère domestique en liaison avec un transfert d'embryons par voie biotechnologique, caractérisé en ce qu'il est constitué d'anticorps neutralisant l'inhibine en liaison avec des hormones favorisant des ovulations multiples (superovulation), de préférence avec des gonadotrophines et/ou avec de la GnRH (hormone libérant de la gonadotrophine) et/ou avec des antioestrogènes sous forme de substances actives synthétiques ou d'anticorps, de préférence de glomiphène ou de tamoxifène et/ou avec des antiandrogènes sous formes de substances actives synthétiques ou d'anticorps, de préférence, à l'encontre de l'androstérone, de la testostérone, de la dihydrotestotérone, ces anticorps neutralisant l'inhibine étant dirigés contre de l'inhibine naturelle de divers types d'animaux et humaine, ou de l'inhibine recombinante (synthétique) ou ses sous-unités ou ses fragments ou ses dérivés, et le médicament pouvant supprimer l'activité biologique de l'inhibine formée dans des gonades et provoquer ainsi, par immunisation dans un cycle de croissance, la croissance folliculaire multiple et la maturation folliculaire multiple jusqu'à la superovulation.

2. Médicament suivant la revendication 1, caractérisé en ce qu'il consiste à utiliser, comme hormones, des gonadotrophines et/ou du GnRH (hormone libérant de la gonadotrophine).

3. Médicament suivant la revendication 1, caractérisé en ce qu'il consiste à utiliser comme hormones des antioestrogènes sous forme de substances actives synthétiques ou d'anticorps.

4. Médicament suivant la revendication 1, caractérisé en ce qu'il consiste à utiliser comme hormones des antiandrogènes sous forme de substances actives synthétiques ou d'anticorps contre les androgènes comme l'androstendione, la testostérone, la dihydrotestostérone.

5. Procédé d'amélioration des réactions ovariennes et de la production d'ovules et d'embryons chez le mammifère domestique en liaison avec un transfert d'embryons par voie biotechnologique en utilisant le médicament suivant l'une des revendications 1 à 4, caractérisé en ce qu'il consiste à effectuer l'application sous la forme d'une injection multiple à des intervalles de temps définis de 12 ou 24 heures sur plusieurs jours successifs, pendant 3,5 jours à 5 jours.

6. Procédé suivant la revendication 5, caractérisé en ce que l'application s'effectue dans la phase lutéinique intermédiaire commençant du septième au onzième jour du cycle.

7. Procédé suivant la revendication 6, caractérisé en ce que l'application s'effectue en des doses égales ou décroissantes avec chaque fois deux quantités égales successives.